# EUROPEAN PATENT APPLICATION

(11) **EP 2 905 019 A1**
(43) Date of publication of application: **12.08.2015**
(21) Application number: 15153775.0
(22) Date of filing: 04.02.2015
(51) Int. Cl.: A61K 9/20, A61K 31/13

(54) **Orally disintegrating tablet formulations of memantine**

(30) Priority: 05.02.2014 TR 201401278
(71) Applicant: Sanovel Ilac Sanayi ve Ticaret A.S., 34460 Istanbul (TR)
(72) Inventor: Türkyilmaz, Ali, 34460 Istanbul (TR); Sezgin, Asiye, 34460 Istanbul (TR); Tombayoglu, Vildan, 34460 Istanbul (TR); Turp, Ali Hasan, 34460 Istanbul (TR)
(74) Representative: Sevinç, Erkan

(57) **Abstract**

The present invention relates to orally disintegrating tablet formulations comprising memantine or a pharmaceutically acceptable salt thereof and one or more pharmaceutically acceptable excipients.

## Description

### Technical Field of the Invention

The present invention relates to orally disintegrating tablet formulations comprising memantine or a pharmaceutically acceptable salt thereof and one or more pharmaceutically acceptable excipients.

### Background of the Invention

Memantine hydrochloride is a neuroprotective medicament, an N-methyl-D-aspartate (NMDA) receptor antagonist, which is efficacious against dementia. Its chemical name is 1,3-dimethyl-5-adamantanamine and its chemical structure is shown in the Formula I.

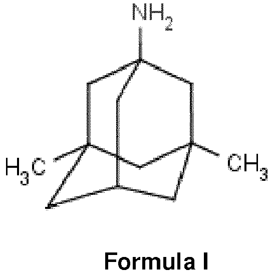

The patent application US3391142 (A) discloses the memantine molecule.

The patent application EP2040676 (A1) discloses an orally dissolving formulation comprising at least one water soluble polymer and memantine or a pharmaceutically acceptable salt thereof.

The patent application EP2211836 (A2) discloses an orally disintegrating tablet composition of memantine or a pharmaceutically acceptable salt thereof comprising taste-masked memantine and at least one pharmaceutically acceptable excipient, wherein the memantine is in complexation with ion exchange resin and the memantine is taste-masked by the ion exchange resin.

In the state of art, there are several patents and applications which disclose orally disintegrating formulations of memantine or pharmaceutically acceptable salts thereof. In the present invention a novel orally disintegrating tablet formulation of memantine or a pharmaceutically acceptable salt thereof is disclosed.

Orally disintegrating formulations are becoming an increasingly important issue in the area of better patient compliance comparative to the conventional solid dosage forms for oral administration such as capsules and tablets, which are the most commonly used. In particular pediatric and geriatric patients and patients with mental problems such as dementia or Alzheimer's disease, often experience difficulties in swallowing solid dosage forms. Besides, conventional solid dosage forms are not suitable for bedridden or busy and travelling patients, in case the patient may not have easy access to water. Thus, orally disintegrating compositions represent an alternative for such patients and provide for a better patient compliance with recommended pharmaceutical therapies.

Additionally oral administration of the drugs is difficult in patients having concomitant vomiting, nausea or diarrhoea. The orally disintegrating dosage form is one of the advantageous methods to deliver the drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of first-pass liver metabolism.

On the other side, orally disintegrating compositions are not easy to process. A satisfied orally disintegrating dosage form needs to meet number of requirements. Firstly, it has to disintegrate in the oral cavity rapidly. Moreover, a premature release in the mouth could also lead to problems due to the often unpleasant taste of the active ingredient. Finally, these compositions should be very porous and should not be very hard. These porous compositions tend to be very sensitive to humidity so they may have some stability problems. Therefore, disintegration, taste and stability are the problems that should be overcome.

### Detailed Description of the Invention

The present invention relates to orally disintegrating tablet formulations comprising memantine or a pharmaceutically acceptable salt thereof is in the range of 1 to 15 %, preferably 5 to 10 % by weight of total formulation and one or more pharmaceutically acceptable excipients.

The object of this invention is to overcome above mentioned problems related to orally disintegrating tablet formulations like disintegration, taste and stability by using appropriate excipients together.
The pharmaceutical formulations according to the present invention may also comprise one or more pharmaceutically acceptable excipient(s). Pharmaceutically acceptable excipients comprise, but are not limited to fillers, disintegrants, glidants, lubricants, sweeteners, aromatic agents, or the mixtures thereof.

In a preferred embodiment of the present invention, said fillers comprise, but are not limited to mannitol, xylitol, trehalose, sorbitol, lactose, sugars, sucrose, inorganic salts, calcium salts, polysaccharides, dextrose, dicalsium phosphate, sodium chloride, dextrates, lactitol, maltodextrin, sucrose-maltodextrin mixtures, heavy magnesium carbonate, izomalt or the mixtures thereof, preferably, it is mannitol, more preferably it is mannitol DC 400.

According to this embodiment, the orally disintegrating tablet formulation of memantine hydrochloride comprises mannitol DC 400 having the average particle size of 360 µm, *Pearlitol 400 DC^{®}.* In this invention, the average particle size is measured by Malvern Particle Size analyzer based on Lazer Diffraction by using dry dispersion method. According to the calculation principle of the analyzer, the volume of the particles is converted into the volume of equivalent sphere and the result is the average diameters of these spheres which is volume moment mean D(4,3) value.

According to this embodiment, the amount of the mannitol DC 400 is in the range of 55 to 85 %, preferably 65 to 75 % by weight of total formulation.

In a preferred embodiment of the present invention, said disintegrants comprise, but are not limited to crospovidon, polyvinylpyrrolidone, alginic acid, alginates, microcrystalline cellulose, sodium starch glycolate, magnesium aluminum silicate, sodium carboxymethyl cellulose, croscarmellose sodium, cross-linked polyvinylpyrrolidone (PVP), vinylpyrrolidone-vinyl acetate copolymers (Kollidon VA64), carboxymethyl cellulose calcium, guar gum, low-substituted hydroxypropyl cellulose, polyacrylin potassium, poloxamer, povidone, sodium alginate, sodium glycine carbonate, ion-exchange resins or the mixtures thereof, preferably it is crospovidone, more preferably it is crospovidon CL-SF.

According to this embodiment, the orally disintegrating tablet formulation of memantine hydrochloride comprises crospovidon CL-SF having the average particle size of 10 to 30 µm, Collidon^{®} CL-SF. In this invention, the average particle size is measured by Malvern Particle Size analyzer based on Lazer Diffraction by using dry dispersion method. According to the calculation principle of the analyzer, the volume of the particles is converted into the volume of equivalent sphere and the result is the average diameters of these spheres which is volume moment mean D(4,3) value.

According to this embodiment, the amount of the crospovidon CL-SF is in the range of 2 to 25 %, preferably 5 to 20 % by weight of total formulation.

In a preferred embodiment of the present invention, crospovidone has physical and chemical properties that make it ideal for constituting the appropriate disintegrant for this invention. Crospovidone particles have a very different appearance from those of the other disintegrants. Crospovidone particles seem to consist of aggregates of smaller particles that are fused together. This aggregation gives crospovidone a spongy, highly porous appearance and it swells very little, yet takes water into its network quite rapidly. This helps crospovidone to dissolve easily and quickly in a little amount of water or saliva and makes its disintegrating rate much faster than other related excipients. Therefore, the composition disintegrates in oral cavity in less than 60 seconds, preferably in less than 30 seconds.

In a preferred embodiment of the present invention, orally disintegrating compositions should be very porous and should not be very hard. These porous compositions tend to be very sensitive to humidity so they may have some stability problems, but it has surprisingly been found that the specific combination of crospovidon CL-SF and mannitol DC 400 creates a synergistic effect over the stability, mechanical strength (such as; hardness and friability) and compressibility of the orally disintegrating tablet formulation.

According to this embodiment, the hardness of the tablets is quite sufficient to allow easy and convenient removal from the package without breaking the dose unit. These orally disintegrating tablets are hard enough to be handled and packaged like conventional tablets. The hardness of the orally disintegrating tablet is between 5 N to 50 N, preferably it is between 10 N to 30 N, and the friability of the orally disintegrating tablet is less than 1.0%.

In a preferred embodiment of the present invention, said glidants comprise, but are not limited to colloidal silicon dioxide, talc, stearic acid, aluminium silicate or the mixtures thereof, preferably, they are colloidal silicon dioxide and talc.

According to this embodiment, the amount of the colloidal silicon dioxide is in the range of 0.1 to 5 %, preferably 0.1 to 3 % by weight of total formulation and the amount of the talc is in the range of 0.5 to 10 %, preferably 1 to 5 % by weight of total formulation.

In a preferred embodiment of the present invention, said lubricants comprise, but are not limited to magnesium stearate, sodium lauryl sulphate, sodium stearyl fumarate, polyethylene glycol, metal stearates, hydrogenated castor oil or the mixtures thereof, preferably, it is magnesium stearate.

According to this embodiment, the amount of the magnesium stearate is in the range of 0.5 to 10 %, preferably 1 to 5 % by weight of total formulation.

In a preferred embodiment of the present invention, said sweeteners comprise, but are not limited to ammonium glycyrrhizinate, sucralose, shaccarin sodium, sucrose, aspartame, glucose, lactose, fructose, other sugars, sorbitol, xylitol, erythritol, other sugar alcohols or the mixtures thereof, preferably, they are ammonium glycyrrhizinate and sucralose.

According to this embodiment, the amount of the ammonium glycyrrhizinate is in the range of 0.01 to 5 %, preferably 0.01 to 2 % by weight of total formulation and the amount of the sucralose is in the range of 0.1 to 5 %, preferably 1 to 3 % by weight of total formulation.

The orally disintegrating compositions of this invention comprise sucralose as a sweetener to improve patient compliance. In prior art, it is know that aspartame is used mostly as sweetner but contradictory to the prior art we have found that the effect of sucralose as a sweetner in this formulation, not only helped to improve its taste but also increased the efficacy and the conveniency of the formulation because of its positive effects over the glycemic index. There are lots of disadvantages about aspartame and it has a limited usage if you have to use it every day and also there are several incompatibilities reported in literature and safety problems *(*Handbook of Pharmaceutical Excipients, Reymond C Rowe, Paul J Sheskey, Marian E Quinn, sixth edition, pages 48-50). Thus, sucralose has an important role in this aspect for orally disintegrating tablet formulations.

Additionally, the orally disintegrating compositions of this invention comprise ammonium glycyrrhizinate as a sweetener in order to obtain the most desirable taste for patients. ammonium glycyrrhizinate is a product derived from licorice root and it has an extremely sweet taste. It provides a consistent taste in the mouth and even very small amount is sufficient to achieve the desired taste. Ammonium glycyrrhizinate can be used in combination with another sweetener since it creates a synergistic effect with both natural and synthetic sweeteners. In this invention it is used with sucralose and it has been surprisingly found that the combination of ammonium glycyrrhizinate and sucralose provides the most pleasant taste for patients.

In a preferred embodiment of the present invention, said aromatic agents comprise, but are not limited to fruit aromas such as orange, banana, strawberry, cherry, wild cherry, lemon, etc., other aromas such as cardamom, anis, mint, menthol, vanillin or the mixtures thereof.

According to this embodiment, the amount of the orange aroma is in the range of 0.5 to 10 %, preferably 1 to 5 % by weight of total formulation.

The formulation according to the present invention may be in the form of a tablet.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time. Therefore, the preferred shape of the orally disintegrating tablet is a round shape.

In this present invention, the formulation has been designed comprising the following:
a) 1 to 15 % by weight of memantine HCl
b) 55 to 85 % by weight of mannitol DC 400
c) 2 to 25 % by weight of crospovidon CL-SF
d) 0.1 to 5 % by weight of colloidal silicondioxide
e) 0.5 to 10 % by weight of talc
f) 0.5 to 10 % by weight of magnesium stearate
g) 0.01 to 5 % by weight of ammonium glycyrrhizinate
h) 0.1 to 5 % by weight of sucralose
i) 0.5 to 10 % by weight of orange aroma

### Example 1 - Orally disintegrating memantine hydrochloride tablets

| **Ingredients** | **Amount (mg)** |
|---|---|
| Memantine HCl | 5.00 |
| Mannitol (Mannitol DC 400) | 56.18 |
| Ammonium glycyrrhizinate | 0.04 |
| Crospovidon CL-SF | 12.00 |
| Sucralose | 1.50 |
| Orange aroma | 2.00 |
| Colloidal silicondioxide | 0.48 |
| Talc | 1.60 |
| Magnesium Stearate | 1.20 |
| **Total weight** | **80** |

The pharmaceutical formulation mentioned above is prepared as following:
a) Memantine HCl, crospovidon CL-SF, mannitol DC 400, sucralose, orange aroma, ammonium glycyrrhizinate, colloidal silicondioxide and talc are sieved by using 1000 µm sieve and mixed for 15 minutes. b) Then, magnesium stearate is sieved by using 1000 µm sieve and added to the mixture (a) and mixed for 2 minutes. The final mixture are compressed as tablets.

### Example 2 - Orally disintegrating memantine hydrochloride tablets

| **Ingredients** | **Amount (mg)** |
|---|---|
| Memantine HCl | 10.00 |
| Mannitol (Mannitol DC 400) | 112.36 |
| Ammonium glycyrrhizinate | 0.08 |
| Crospovidon CL-SF | 24.00 |
| Sucralose | 3.00 |
| Orange aroma | 4.00 |
| Colloidal silicondioxide | 0.96 |
| Talc | 3.20 |
| Magnesium Stearate | 2.40 |
| **Total weight** | **160** |

The pharmaceutical formulation mentioned above is prepared as following:
a) Memantine HCl, crospovidon CL-SF, mannitol DC 400, sucralose, orange aroma, ammonium glycyrrhizinate, colloidal silicondioxide and talc are sieved by using 1000 µm sieve and mixed for 15 minutes. b) Then, magnesium stearate is sieved by using 1000 µm sieve and added to the mixture (a) and mixed for 2 minutes. The final mixture are compressed as tablets.

### Example 3 - Orally disintegrating memantine hydrochloride tablets

| **Ingredients** | **Amount (mg)** |
|---|---|
| Memantine HCl | 15.00 |
| Mannitol (Mannitol DC 400) | 168.54 |
| Ammonium glycyrrhizinate | 0.12 |
| Crospovidon CL-SF | 36.00 |
| Sucralose | 4.50 |
| Orange aroma | 6.00 |
| Colloidal silicondioxide | 1.44 |
| Talc | 4.80 |
| Magnesium Stearate | 3.60 |
| **Total weight** | **240** |

The pharmaceutical formulation mentioned above is prepared as following:
a) Memantine HCl, crospovidon CL-SF, mannitol DC 400, sucralose, orange aroma, ammonium glycyrrhizinate, colloidal silicondioxide and talc are sieved by using 1000 µm sieve and mixed for 15 minutes. b) Then, magnesium stearate is sieved by using 1000 µm sieve and added to the mixture (a) and mixed for 2 minutes. The final mixture are compressed as tablets.

### Example 4 - Orally disintegrating memantine hydrochloride tablets

| **Ingredients** | **Amount (mg)** |
|---|---|
| Memantine HCl | 20.00 |
| Mannitol (Mannitol DC 400) | 224.72 |
| Ammonium glycyrrhizinate | 0.160 |
| Crospovidon CL-SF | 48.00 |
| Sucralose | 6.00 |
| Orange aroma | 8.00 |
| Colloidal silicondioxide | 1.92 |
| Talc | 6.40 |
| Magnesium Stearate | 4.80 |
| **Total weight** | **320** |

The pharmaceutical formulation mentioned above is prepared as following:
a) Memantine HCl, crospovidon CL-SF, mannitol DC 400, sucralose, orange aroma, ammonium glycyrrhizinate, colloidal silicondioxide and talc are sieved by using 1000 µm sieve and mixed for 15 minutes. b) Then, magnesium stearate is sieved by using 1000 µm sieve and added to the mixture (a) and mixed for 2 minutes. The final mixture are compressed as tablets.

### Example 5 - Hardness and disintegration time of the orally disintegrating memantine hydrochloride tablets for examples 1 to 4

According to standardized methods and equipment for testing **hardness** and disintegrating time have been provided in European Pharmacopoeia. These orally disintegrating tablet formulations of the invention (Ex.1 to 4) are tested according to these methods.

| **Examples** | **Hardness (Newton)** | **Disintegration time (sec)** |
|---|---|---|
| **1** | 23 | 6 |
| **2** | 26 | 9 |
| **3** | 26 | 11 |
| **4** | 16 | 15 |

### Example 6 - Friability (%) of the orally disintegrating memantine hydrochloride tablets for examples 1 to 4

According to standardized methods and equipment for testing friability (%) have been provided in European Pharmacopoeia. These orally disintegrating tablet formulations of the invention (Ex.1 to 4) are tested according to these methods.

| **Examples** | **Friability (%) of sample 1** | **Friability(%)of sample 2** |
|---|---|---|
| **1** | % 0.10 | % 0.22 |
| **2** | % 0.26 | % 0.21 |
| **3** | % 0.28 | % 0.42 |
| **4** | % 0.8 | % 0.8 |

## Claims

1. An orally disintegrating tablet formulation comprising memantine or a pharmaceutically acceptable salt thereof is in the range of 1 to 15 % by weight of total formulation.

2. The orally disintegrating tablet formulation according to claim 1, wherein one or more pharmaceutically acceptable excipient is selected from the group comprising fillers, disintegrants, glidants, lubricants, sweeteners, aromatic agents, or the mixtures thereof.

3. The orally disintegrating tablet formulation according to any of the preceding claims, wherein the filler is selected from the group comprising mannitol, xylitol, trehalose, sorbitol, lactose, sugars, sucrose, inorganic salts, calcium salts, polysaccharides, dextrose, dicalsium phosphate, sodium chloride, dextrates, lactitol, maltodextrin, sucrose-maltodextrin mixtures, heavy magnesium carbonate, izomalt or the mixtures thereof, preferably, it is mannitol.

4. The orally disintegrating tablet formulation according to any of the preceding claims, wherein the amount of mannitol is in the range of 55 to 85 %, preferably 65 to 75 % by weight of total formulation.

5. The orally disintegrating tablet formulation according to any of the preceding claims, wherein the disintegrant is selected from the group comprising crospovidone, polyvinylpyrrolidone, alginic acid, alginates, microcrystalline cellulose, sodium starch glycolate, magnesium aluminum silicate, sodium carboxymethyl cellulose, croscarmellose sodium, cross-linked polyvinylpyrrolidone (PVP), vinylpyrrolidone-vinyl acetate copolymers (Kollidon VA64), carboxymethyl cellulose calcium, guar gum, low-substituted hydroxypropyl cellulose, polyacrylin potassium, poloxamer, povidone, sodium alginate, sodium glycine carbonate, ion-exchange resins or the mixtures thereof, preferably it is crospovidon.

6. The orally disintegrating tablet formulation according to any of the preceding claims, wherein the amount of crospovidon is in the range of 2 to 25 %, preferably 5 to 20 % by weight of total formulation.

7. The orally disintegrating tablet formulation according to any of the preceding claims, wherein the glidant is selected from the group comprising colloidal silicon dioxide, talc, stearic acid, aluminium silicate or the mixtures thereof, preferably, they are colloidal silicon dioxide and talc.

8. The orally disintegrating tablet formulation according to any of the preceding claims wherein the amount of colloidal silicon dioxide is present in the range of 0.1 to 5 %, preferably 0.1 to 3 % by weight of total formulation and the amount of the talc is in the range of 0.5 to 10 %, preferably 1 to 5 % by weight of total formulation.

9. The orally disintegrating tablet formulation according to any of the preceding claims, wherein the lubricant is selected from the group comprising magnesium stearate, sodium lauryl sulphate, sodium stearyl fumarate, polyethylene glycol, metal stearates, hydrogenated castor oil or the mixtures thereof, preferably, it is magnesium stearate.

10. The orally disintegrating tablet formulation according to any of the preceding claims, wherein the amount of magnesium stearate is in the range of 0.5 to 10 %, preferably 1 to 5 % by weight of total formulation.

11. The orally disintegrating tablet formulation according to any of the preceding claims, wherein the sweetener is selected from the group comprising ammonium glycyrrhizinate, sucralose, shaccarin sodium, sucrose, aspartame, glucose, lactose, fructose, other sugars, sorbitol, xylitol, erythritol, other sugar alcohols or the mixtures thereof, preferably, they are ammonium glycyrrhizinate and sucralose.

12. The orally disintegrating tablet formulation according to any of the preceding claims, wherein the amount of ammonium glycyrrhizinate is present in the range of 0.01 to 5 %, preferably 0.01 to 2 % by weight of total formulation and the amount of the sucralose is in the range of 0.1 to 5 %, preferably 1 to 3 % by weight of total formulation.

13. The orally disintegrating tablet formulation according to any of the preceeding claims, wherein the composition disintegrates in oral cavity in less than 60 seconds, preferably in less than 30 seconds.

14. The orally disintegrating tablet formulation according to any of the preceding claims, wherein the hardness of the tablet is between 5 N to 50 N, preferably it is between 10 N to 30 N.

15. The orally disintegrating tablet formulation according to any of the preceding claims comprising;
a) 1 to 15 % by weight of memantine HCl
b) 55 to 85 % by weight of mannitol
c) 2 to 25 % by weight of crospovidon
d) 0.1 to 5 % by weight of colloidal silicondioxide
e) 0.5 to 10 % by weight of talc
f) 0.5 to 10 % by weight of magnesium stearate
g) 0.01 to 5 % by weight of ammonium glycyrrhizinate
h) 0.1 to 5 % by weight of sucralose
i) 0.5 to 10 % by weight of orange aroma
